# EUROPEAN PATENT SPECIFICATION

(11) **EP 2 039 758 B1**
(45) Date of publication and mention of the grant of the patent: **01.09.2010**
(21) Application number: 07018574.9
(22) Date of filing: 21.09.2007
(51) Int. Cl.: C12N 5/02, C12N 5/0789

(54) **Cell culture medium and method for culturing stem cells and progenitor cells**
Zellkulturenmedium und Verfahren zur Kultivierung von Stammzellen und Vorläuferzellen
Support de culture de cellule et procédé de culture de cellules souches et cellules de progéniteur

(43) Date of publication of application: 25.03.2009
(73) Proprietor: Universität Leipzig, 04109 Leipzig (DE)
(72) Inventor: Cross, Michael, Dr., 04299 Leipzig (DE); Alt, Rüdiger, 06110 Halle/Saale (DE)
(74) Representative: Stötter, Gerd

(56) References cited:
- US-A- 5 747 341
- US-A1- 2005 221 483
- STOLZING A ET AL: "Glucose-induced replicative senescence in mesenchymal stem cells" REJUVENATION RESEARCH 2006 UNITED STATES, vol. 9, no. 1, 2006, pages 31-35, XP009092721 ISSN: 1549-1684
- LENNON D P ET AL: "A chemically defined medium supports in vitro proliferation and maintains the osteochondral potential of rat marrow-derived mesenchymal stem cells" EXPERIMENTAL CELL RESEARCH 1995 UNITED STATES, vol. 219, no. 1, 1995, pages 211-222, XP000981537 ISSN: 0014-4827
- TENNANT G B ET AL: "Control of pH in human long-term bone marrow cultures with low-glucose medium containing zwitterion buffer lengthens the period of haemopoietic activity" BRITISH JOURNAL OF HAEMATOLOGY 2000 UNITED KINGDOM, vol. 109, no. 4, 2000, pages 785-787, XP002459995 ISSN: 0007-1048

## Description

The invention relates to the use of a cell culture medium, which allows extensive expansion of somatic stem cells such as haematopoietic stem cells and/or their early progenitor cells and germ line stem cells, while maintaining their regenerative potential and a method for culturing stem cells and a method for culturing stem cells and/or their early progenitor cells.

*Technical field*: Stem cells are primal cells found in most multi-cellular organisms. They retain the ability to renew themselves through mitotic cell division and can differentiate into a diverse range of specialized cell types. The three broad categories of mammalian stem cells are: embryonic stem cells, derived from blastocysts; germ line stem cells, responsible for generating gametes; and somatic stem cells. The term somatic or adult stem cell refers to any cell other than an embryonic stem cell and other than a germ line stem cell and that has two properties: the ability to divide and create another cell like itself and also divide and create a cell more differentiated than itself. The term somatic stem cell is preferred over the term adult stem cell, as the cells can be found during development and in children, as well as in adults. Most somatic stem cells can generate a restricted number of lineages (multipotent) and may be referred to by their tissue of origin (e.g. bone marrow stem cells) or by their differentiation potential (hematopoietic stem cell, mesenchymal stem cell, endothelial stem cell, etc.). Hematopoietic stem cells (HSC) are somatic stem cells, which can be isolated from the bone marrow or the blood, including umbilical cord blood. HSC are able to regenerate HSC, and to differentiate into all the blood cell types including the myeloid (monocytes and macrophages, neutrophils, basophils, eosinophils, erythrocytes, megakaryocytes/platelets and some dendritic cells) and lymphoid lineages (T-cells, B-cells, NK-cells, some dendritic cells). Hematopoietic progenitor cells are derived from HSC and have lost the ability to make one or more of these lineages, but are not yet mature. The term "early progenitors" refers to those cells most directly derived from the HSC, which retain most or all of the differentiation potential but have lost the ability to regenerate HSC. In an experimental setting, HSC retain the ability to regenerate a functional hematopoietic system indefinitely (long term repopulating activity), while early progenitors have the ability to regenerate a damaged hematopoietic system for a period of a few (usually 3) months (short term repopulating activity).

To ensure self-renewal, stem cells undergo two types of cell division. Symmetric division gives rise to two identical daughter cells both endowed with stem cell properties. Asymmetric division, on the other hand, produces only one stem cell and a progenitor cell with limited self-renewal potential.

Cell strains are cells adapted to cell culture, but with finite division potential. A cell line is a permanently established cell culture that will proliferate indefinitely given appropriate fresh medium and space. Cell lines differ from cell strains in that they have escaped the Hayflick limit and become immortalised. The term cells used in the following text and the claims does include primary cells, cell strains and cell lines.

Hematopoietic stem cells contained in cell fractions isolated from bone marrow or blood have been used for many years to generate a functional blood and immune system in patients whose own system has been damaged by chemotherapy, disease or radiation exposure. However, the low frequency and mitotic inactivity typical of tissue stem cells remains a significant barrier both to analysis and to clinical application. Hence, the numbers of therapeutic stem cells in a single umbilical cord blood are usually considered insufficient to transplant an adult patient, while the genetic modification of stem cells in culture for the purposes of gene therapy is limited both by their low frequency and low mitotic activity compared to differentiating progenitors. Similarly, the low numbers of hematopoietic stem cells preclude attempts to generate material either for repeated transplantation of HSCs or for reprogramming into therapeutically useful volumes of non-haematopoietic tissues.

*State of the Art*: For these reasons, there have been many attempts to establish culture conditions under which hematopoietic stem cells can be expanded (Hofmeister et al., 2007). These include culture in direct contact with supportive (stromal) cells; the use of media conditioned by supportive cells; and the supplementation of media with a variety of substances which favour the survival and proliferation of undifferentiated cells.

The media employed in the culture of haematopoietic stem cells to date are based on adaptation and supplementation of standard basal media (including Eagle's basal medium, Dulbecco's modified Eagle's medium, Iscove's modified Dulbecco's medium, Fischer's Medium and Glasgow medium), which contain both glucose (generally at concentrations of between 1 g/L and 4.5 g/L) and inositol (generally in the form *myo*-inositol, *cis*-1,2,3,5-*trans-*4,6-cyclohexanehexol, at concentrations between 1mg/L and 36 mg/ml). Minimum Essential Medium Eagle (MEM) contains 1 g/L glucose and 2 mg/L Inositol.

Glucose is a major source of carbon and energy and is generally assumed to be necessary for the culture of all mammalian cells with the exception of a few cell types that are capable of synthesising their own glucose by gluconeogenesis. Media referred to as low glucose generally contain lg/litre glucose, while those referred to as high glucose generally contain 4.5 g/L glucose. Inositol is an essential constituent of a family of molecules (phosphatidylinositol and the inositol phosphates) which are necessary for the communication of signals from a variety of receptors for extracellular regulators of cell survival, proliferation and differentiation. Although inositol can be synthesised intracellularly from glucose, it is common practice to include inositol in media. The concentrations normally used vary widely between 0.05 mg/L and 36 mg/L. A commercially available medium intended explicitly for the culture of hematopoietic stem and progenitor cells (Methostem, PAA Laboratories) contains 4.5 g/L glucose and 7.2 mg/L inositol as myo-inositol).

US 5,747,341 discloses cell culture media having low osmolarity for establishing and maintaining hormone-secreting cells in cell culture. Formula VI with a glucose content of 360 mg/L is intended specifically for a short period of glucose starvation in pancreatic cell cultures in order to test the subsequent response to glucose.

Stolzing et al. 2006 (Rejuvenation Research 9(1): 31 - 35) describe the glucose-induced replicative senescence in mesenchymal stem cells. The effect of glucose concentrations between 250 mg/L up to 4500mg/L was examined.

*Deficiencies of known solutions:* In none of the media formulations available to date has it been possible to achieve extensive expansion of hematopietic stem cells (HCS) without a decrease in their regenerative potential, stem cell division under the culture conditions described to date being closely associated with progressive differentiation (Wilson and Trumpp, 2006).

The first objective of the invention is to provide a cell culture medium, which allows culturing and expansion of somatic stem cells or germ line stem cells and their early progenitor cells, in particular hematopoietic stem and their early progenitor cells, while maintaining their regenerative potential. A second objective of the invention is a method for culturing, amplifying and/or enriching stem cells, in particular hematopoietic stem cells and/or their progenitor cells, using the cell culture medium of the invention.

The first objective is solved according to claim 1 by the use of a stem cell culture medium that is deficient in both glucose and inositol.

The novel solution to this problem is based on our recognition and characterisation of the unusual metabolic activity of somatic stem cells and germ line stem cells, in particular hematopoietic stem cells. Our own research has shown that, while most cells die rapidly as expected in media deficient in both glucose and inositol, hematopoietic stem cells are capable of survival and growth under these conditions. This shows that metabolic and signalling pathways in haematopoietic stem cells differ markedly from those in their more differentiated progeny. In short, while differentiated cells use substantial amounts of glucose (for metabolism) and inositol (for the propagation of receptor-mediated signals influencing survival and proliferation), the requirement for glucose and inositol in HSCs is low or absent. It is relevant here that many growth factors which support differentiating progenitors, leukaemic cells or mature cells in vitro activate inositol-dependent signalling pathways, while some of the growth factors which provide specific support HSC in vitro (such as those activating the Wnt, Notch and Hedgehog pathways) are largely or wholly independent of inositol. Rather, the presence of glucose and inositol at levels functional in metabolism and signalling supports stem cell differentiation and leads to an accumulation of cells which have lost stem cell potential. This invention provides a previously unknown means for selectively isolating, maintaining and proliferating HSCs.

Thus, according to the invention the amount of glucose and inositol in the cell culture medium is kept below levels required for the production of differentiating cells.

The cell culture medium according to the invention preferably consists of a basal medium, which can be provided either as liquid, as stock solution or in form of a powder, whereas the stock solution or powder are diluted with sterile water before application. The basal medium is essentially free of glucose and inositol, meaning that it contains less than 10 mg glucose/L and less than 10 µg Inositol /L in the diluted liquid.

The basal medium deficient in glucose and inositol preferably contains other normal ingredients of a standard basal medium including inorganic salts, a buffer system, amino acids, fatty acids/lipids, pyruvate and vitamins, nucleosides, a pH indicator and a biocompatible reducing agent. However, the inclusion of glutamate (to a final concentration of 10 - 500 mg/L) is preferred to glutamine in order to allow for the elimination of excess amino groups (through the conversion of glutamate to glutamine) resulting from the catabolism of amino acids in the absence of metabolic levels of glucose. Furthermore, the final concentration of each nucleoside is preferably below 1 mmol/L.

The basal medium can advantageously be stored for months. Before the application the basal medium is preferably supplemented by serum (e. g. from bovine, horse or human) or a serum replacement component. In a preferred embodiment autologous serum is used. The serum is preferably dialysed to reduce the glucose and inositol content. The serum is preferably be added to the medium to a final content of 0.5 % to 30 %, preferably 1 to 20 %, most preferably 5-10%.

The serum replacement preferably contains the serum proteins albumin and transferrin (preferably saturated with iron) selenium and essential fatty acids, like C₁₆-C₂₄ unsaturated fatty acids. It preferably does not contain detectable Ig and mitogens. Commercially available serum replacemnts can be used if the glucose and inositol content are low enough. The proteins in the serum replacement are either isolated from natural source (e. g. from bovine, horse or human) or produced recombinant.

The serum or serum replacement is preferably heat-inactivated.

Further possible supplements in the basal medium or the cell culture medium include albumin, selenium, transferrin, essential fatty acids, glutamate, lactate, glutamine, biocompatible reducing agents (such as glutathione or mercaptoethanol), growth factors and other modulators (activators or inhibitors) of cellular signalling pathways.

Preferred growth factors and modulators of cellular signalling pathways to be added to the medium are factors that support stem cell proliferation via inositol-independent pathways. Most preferred growth factors are selected from factors of the Wnt/Wingless, Notch ligand, Hedgehog and angiopontin families, as well as interleukin-3, interleukin-6, stem cell factor, thrombopoietin, FLT3-L (fetal liver tyrosine kinase like 3 ligand). The growth factors are either isolated from natural source or preferably produced recombinant. Examples of modulators of cell signalling pathways are: cyclic ADP ribose (cADPR); Hedgehog agonist (Hh-Ag) and pharmacological inhibitors of glycogen synthase kinase 3 activity.

According to the invention the glucose and inositol content of the serum or other components to be supplemented is also kept very low. This low glucose and inositol content of the serum is preferably achieved by dialysis and/or enzymatic degradation of glucose.

Due to the low glucose and inositol content of the basal medium, the serum (or serum replacement) and all possible further supplements it is secured that the final medium used for cell culturing contains less than 500 mg glucose/L, preferably less than 100 mg glucose/L, most preferably less than 10 mg glucose /L and less than 500 µg inositol /L, preferably less than 100 µg inositol /L, most preferably less than 10 µg inositol /L.

Further the basal medium, as well as the cell culture medium contains less than 500 mg/L, preferably less than 100 mg/L, most preferably less than 10 mg/L, of other sugars than glucose (such as fructose, mannose or galactose) which are catabolised by the glycolytic pathway. The medium preferably also does contain less than 500 µg/L, preferably less than 100 µg/L, most preferably less than 10 µg, inositol equivalents in inositol containing substances, as inositol phosphates, phosphatidylinositol (PI) and phosphatidylinositol phosphate (PIP) lipids.

Preferred relative amounts (referring to the final dilution) of the various components of the basal medium are listed in the following. As most of these components are also included in the serum, the amounts necessary in the basal medium often depend on the serum content of the final cell culture medium.

The basal medium preferably contains a combination of salts generally present in standard basal media to provide a buffer system and a source of the following ions: sodium, chloride, potassium, magnesium, calcium, chlorine, phosphor, sulphur, selenium and preferably zinc, iron, copper.

Preferred salts and their preferred concentrations in the basal medium (final dilution) are selected from the following list:

| | |
|---|---|
| Calcium chloride (preferably CaCl₂.2H₂O) | between 1 mg/L and 300 mg/L preferably between 100 mg /L and 250 mg/L |
| Potassium chloride (KCl) | between 200 mg/L and 600 mg/L preferably between 250 mg /L and 400 mg/L |
| Magnesium sulfate (preferably MgSO₄.7H₂O) | between 50 mg/L and 400 mg/L preferably between 150 mg /L and 300 mg/L |
| Sodium chloride (NaCl) | between 4000 mg/L and 10000 mg/L |
| | preferably between 4500 mg /L and 6000 mg/L |
| Sodium hydrogen carbonate (NaHCO₃) | between 300 mg/L and 5000 mg/L |
| | preferably between 2000 mg /L and 3000 mg/L |
| Sodium dihydrogen phosphate | between 50 mg/L and 1000 mg/L |
| (preferably NaH₂PO₄.2H₂O) | preferably between 100 mg /L and 300 mg/L |
| Iron itrate (preferably FeNO₃.9H₂O) | between 0 mg/L and 2 mg/L |
| Zinc sulfate (preferably ZnSO₄.7H₂O) | between 0 mg/L and 2 mg/L |
| Copper sulphate (preferably CuSO₄.5H₂O) | between 0 mg/L and 0.1 mg/L |
| Sodium hydrogen carbonate (NaHCO₃) to provide a buffering system in combination with CO₂ supplied in the atmosphere. | between 300 mg/L and 5000 mg/L |
| HEPES (4-(2-hydroxyethyl)-1-piperazineethanesulfonic acid ) to provide an additional buffering system. | between 0 g/L and 10 g/L |

The basal medium further contains amino acids included in standard basal media. However a low concentration or absence of glutamine combined with a high concentration of glutamate is preferred to provide an acceptor of excess amino groups generated by amino acid catabolism. The basal medium preferably contains less than 200 mg/L, more preferably less than 50 mg/L, L-glutamine and between 10 mg/L and 500 mg/L, preferably 100 mg/L to 400 mg/L, L-glutamate (referring to the final diluted solution).

Further a high concentration of serine, together with either or both of glycine and cystein is preferred. The basal medium preferably contains between 10 mg/L and 200 mg/L L-serine preferably between 20 mg/L and 50 mg/L-serine,and between 5 mg/L and 100 mg/L-glycin preferably between 10 and 50 mg/L L-glycine and/or between 5 mg/L and 200 mg/L L-cysteine preferably between 20 and 50 mg/L L-cysteine.

Preferred amino acids and their preferred concentrations in the basal medium (final dilution) are selected from the following list:

| | |
|---|---|
| L-alanine | between 5 mg/L and 50 mg/L |
| L-asparagine | between 5 mg/L and 20 mg/L |
| L-arginine | between 10 mg/L and 200 mg/L |
| L-aspartate | between 5 mg/L and 50 mg/L |
| L-cysteine | between 5 mg/L and 200 mg/L |
| L-glutamate | between 10 mg/L and 500 mg/L |
| L-glutamine | between 0 mg/L and 200 mg/L |
| Glycin | between 5 mg/L and 100 mg/L |
| L-hisitidine | between 5 mg/L and 100 mg/L |
| L-isoleucine | between 10 mg/L and 150 mg/L |
| L-leucine | between 10 mg/L and 150 mg/L |
| L-lysine | between 10 mg/L and 150 mg/L |
| L-methionine | between 5 mg/L and 150 mg/L |
| L-phenylalanine | between 10 mg/L and 100 mg/L |
| L-proline | between 5 mg/L and 50 mg/L |
| L-serine | between 10 mg/L and 200 mg/L |
| L-threonine | between 10 mg/L and 100 mg/L |
| L-tryptophan | between 2 mg/L and 20 mg/L |
| L-tyrosine | between 10 mg/L and 100 mg/L |
| L-valine | between 10 mg/L and 100 mg/L |

The basal medium preferably further contains vitamins. Preferred vitamins and their preferred concentrations in the basal medium (final dilution) are selected from the following list:

| | |
|---|---|
| Pantothenate | between 0 mg/L and 15mg/L |
| | preferably between 2 and 6 mg/L |
| Choline | between 0 mg/L and 50 mg/L |
| | preferably between 2 and 6 mg/L |
| Folic acid | between 0 mg/L and 20 mg/L |
| | preferably between 2 and 6 mg/L |
| Nicotinamide | between 0 mg/L and 20 mg/L |
| | preferably between 2 and 6 mg/L |
| Pyridoxin | between 0 mg/L and 10 mg/L |
| | preferably between 2 and 6 mg/L |
| Riboflavin | between 0 mg/L and 2 mg/L |
| | preferably between 0.05 and 0.5 mg/L |
| Thiamine | between 0 mg/L and 10 mg/L |
| | preferably between 2 and 6 mg/L |
| Vitamin B₁₂ | between 0 and 5 mg/L, |
| | preferably between 0.01 mg/L and 2 mg/L |
| Vitamin E (Tocopherol) | between 0 mg/L and 10 mg/L |
| | preferably between 2 and 6 mg/L |

The basal medium preferably contains nucleosides between 1 mg/L and 1 g/L, preferably between 1 mg/L and 50mg/L each of adenosine, guanosine, cytidine and uridine (referring to the final diluted solution).

Preferred further components and their concentrations in the basal medium (final dilution) are selected from the following list:

| | |
|---|---|
| pH-Indicator, as Phenol red | between 0 mg/L and 20 mg/L |
| Pyruvate | between 0 mg/L and 550 mg/L |
| Lactate | between 0 mg/L and 550 mg/L |
| Linoleic acid | between 0 mg/L and 1 mg/L |
| Linolenic acid | between 0 mg/L and 1 mg/L |
| Non-essential fatty acids | between 0 mg/L and 10 mg/L |
| Cholesterol | between 0 mg/L and 100 mg/L |
| Lipoic acid | between 0 mg/L and 5 mg/L |

Prior to use, the medium is preferably supplemented with serum from human, bovine or equine sources, to a final content of between 0.5% and 30 % v/v, preferably 1 to 20 % v/v, most preferably 5-10% v/v. Before use, the glucose and inositol content of the serum is reduced, preferably by dialysis, so that the final medium used for cell culturing contains less than 500 mg glucose/L, preferably less than 100 mg glucose/L, most preferably less than 10 mg glucose /L and less than 500 µg inositol /L, preferably less than 100 µg inositol /L, most preferably less than 10 µg inositol /L.

The basal medium or the cell culture medium is preferably additionally supplemented with one or more of the following components:

| | |
|---|---|
| Albumin (preferably essentially free of fatty acid and globulin) preferably from human, bovine, porcine, equine or recombinant sources | between 0.1 g/L and 10g/L |
| Sodium selenite (NaSeO₃) | between 0.005 mg/L and 0.05 mg/L |
| Transferrin | between 0.01 mg/L and 1 mg/L |
| Glutathione | between 0.001 mg/L and 2 mg/L |
| Mercaptoethanol | between 1µM and 500µM |
| Glutamine | between 0 g/L and 200 g/L |

The medium may also be supplemented with biologically active concentrations of growth factors and other activators or inhibitors of cellular signalling pathways (examples are mentioned above).

The combination and concentration of components is chosen to result in an osmolarity of the basal medium between 0.28 and 0.4 Osm/Kg before supplementation. The osmolarity of the supplemented medium should be adjusted to between 0.32 and 0.36 Osm/Kg, preferably 0.36 Osm/Kg before use. Osmolarity is normally adjusted either by dilution with sterile water (to reduce osmolarity) or by adding sodium chloride to increase osmolarity.

The cell culture medium according to the invention is used for culturing somatic stem cells or germ line stem cells (such as spermatagonial stem cells) including primary cells, cells strains or cell lines of the above mentioned stem cells.

Somatic stem cells include in particular hematopoietic stem cells and early progenitor cells, as well as epithelial stem cells (in particular from skin or gut) and adipose-derived stem cell.

The key advantage of the stem cell culture medium according to the invention is that it allows culturing and expansion without unwanted differentiation of the stem cells, maintaining their stem cell characteristics.

The medium according to the invention can in particular be used for:
1. the purification of somatic stem cells or germ line stem cells (enrichment of stem cells away from other cell types);
2. the culturing of somatic stem cells or germ line stem cells retaining their stem cell function;
3. the amplification of somatic stem cells or germ line stem cells retaining their stem cell function;
4. the purification of non-leukaemic stem cells from leukaemic cells in blood or bone marrow from a patient with leukaemia;
5. the culturing of somatic stem cells or germ line stem cells maintaining the genetic integrity of the cells.

Another object of the invention is a method for culturing, amplifying, purifying and/or enriching somatic stem cells or germ line stem cells, wherein cells are cultured in a cell culture medium the invention or, preferably in an atmosphere containing 0,1% to 5% O₂(v/v). The atmosphere preferably further contains 4 to 6 %, CO₂. The rest being a biochemically inert gas like N₂.

The invention involves also a method for purification of somatic stem cells or germ line stem cells (enrichment of stem cells away from other cell types) by culturing a stem cell containing cell mixture, derived for example from blood or bone marrow, into the medium of the invention. The enrichment of these stem cells is achieved due to the fact that most non-stem cells do depend on glucose as a source of energy and on inositol (which may be derived from glucose) to transmit receptor-mediated signals supporting survival, proliferation and differentiation. Thus, they will not grow in the medium according to the invention.

This method preferably involves a preliminary enrichment using one of a number of methods in general use, then introduction of the cell mixture into the medium as defined in the invention deficient in glucose and inositol. This preliminary enrichment is preferably achieved in two stages. In the first stage, a white blood cell fraction containing the stem cells is separated from other blood components either by hypotonic lysis of erythrocytes followed by centrifugation to collect the remaining cells, or by buoyant density centrifugation to separate the mononuclear cell fraction (containing stem cells). In the second stage, stem cells are further enriched from these white blood cell populations by the binding of antibodies which are selective either for stem cell-containing populations (eg antibodies to CD133 and/or CD34) or for non-stem cell populations (eg antibodies to CD38 and/or lineage markers). Specific cell populations identified in this way are isolated either by fluorescent activated cell sorting (FACS, requiring the use of fluorescent antibodies) or magnetic affinity cell sorting (MACS). The identification and isolation of stem-cell enriched populations by FACS may also be achieved by the use of fluorescent dyes (such as Rhodamine 123 and/or Hoechst 33342) or by the use of fluorescent substrate for an enzyme (eg aldehyde dehydrogenase) which show differential staining characteristics in stem cell enriched and stem cell depleted populations. The preliminary enrichment may involve a combination of these approaches. Following a period of culture from 12 hours to 1 week, normally 3 days, the surviving cell fraction is highly enriched for stem cells. This enrichment is in particular due to the fact that most non-stem cells do depend on glucose as a source of energy and on inositol (which may be derived from glucose) to transmit receptor-mediated signals supporting survival, proliferation and differentiation. Thus, they will not grow but will die in the medium according to the invention.

The invention involves also a method for amplification of somatic stem cells or germ line stem cells that retain stem cell function. The medium according to the invention supports symmetric division rather than asymmetric amplification of stem cells. Stem cell enriched cell fractions, preferably obtained with the methods described above, are introduced into the medium according to the invention (deficient in glucose and inositol) which is supplemented with activators of stem cell growth (examples being growth factors of the Wnt, Notch or Hedgehog families, or substances which mimic their effects). Long term culture over periods in excess of 1 week, normally 4-12 weeks, supports amplification of the stem cell population without the accumulation of differentiating cells.

The invention involves also a method for purification of non-cancerous somatic stem cells or germ line stem cells from cancer cells, in particular non-cancerous stem cells in a blood or bone marrow from a patient with cancer, particularly leukaemia. This is achieved by applying the methods described above to cells derived from the bone marrow or blood of the patient. By culturing the cells in the medium according to the invention the material will be depleted for leukaemic cells while retaining normal stem cell function. This effect results from the fact that cancerous (and particularly leukaemic) transformation results in part from mutations which activate signalling pathways dependent on glycolysis and on inositol-dependent signalling. Cancerous stem and progenitor cells are then expected to be more dependent on glucose and inositol than are non-cancerous stem cells. Culture of a mixture of cancerous and non-cancerous stem cells in the medium of the invention will therefore result in enrichment of the non-cancerous stem cells and depletion of the cancerous cells. This is referred to as "purging" the cancerous cells.

In each case, the enriched or amplified cell fractions are available for experimental analysis; further culture under conditions which support the development of haematopoietic or other cell types (for instance in tissue engineering applications); genetic manipulation; and/or transplantation into patients. The cell fraction purged of cancer cells provides a source of stem cells for autologous transplantation of cancer patients, in particular leukaemia patients.

Advantages of the invention are summarized as follows:
Advantage 1: differentiated cell types are dependent on inositol-mediated signalling for survival. A medium deficient in both inositol and glucose therefore permits the enrichment by selective survival of somatic stem cells or germ line stem cells from mixtures of stem and non-stem cells. (Major application: providing enriched stem cell fractions for research and therapy)
Advantage 2: In the absence of glucose and inositol, leading to the absence of inositol-dependent signalling, somatic stem cells or germ line stem cells cannot generate viable differentiated cells. When supplemented with the growth factors supporting stem cell proliferation via inositol-independent pathways, the medium permits amplification of stem cells without loss of potential. (Major application - increasing the number of stem cells available for transplantation, gene therapy, tissue engineering and research purposes)
Advantage 3: cancer cells typically have a predominantly glycolytic metabolism, and are therefore incapable of survival and proliferation in glucose-free medium, providing a basis for the selective purification of normal somatic stem cells or germ line stem cells from cancerous tissue. (Application purging leukaemic bone marrow for the generation of autologous transplants)
Advantage 4: Culturing under the conditions described will be important not just for amplifying somatic stem cells or germ line stem cells, but also for maintaining the genetic integrity of the cells. In a standard cell culture condition in which glucose is abundant, the energy level of the cells is high and inositol is plentiful, any mutation which increases the signals for proliferation of a cell bestows on that cell a selective advantage, so that the descendants of the mutated cell (which also carry the mutation) progressively overgrow the other cells. This provides a basis for the progressive selection of increasingly cancerous cells in cell culture media known from the state of the art. In the medium of the invention the energy levels and inositol availability are low, so that the rate of proliferation is limited more by metabolic activity and less by signalling. Mutations which potentially increase proliferative signals therefore have little effect. For this reason there will therefore be less, if any, selection for these potentially cancerous mutations.

The invention is illustrated by the following examples without being limited to these:

### Example 1: Example for a medium composition

A suitable basal medium comprises:

| | |
|---|---|
| Calcium chloride (CaCl₂.2H₂O) | 218 mg /L |
| Potassium chloride (KCl) | 330 mg/L |
| Magnesium sulfate (MgSO₄.7H₂O) | 200 mg/L |
| Sodium chloride (NaCl) | 4.5 g/L |
| Sodium hydrogen carbonate (NaHCO₃) | 2.7 g/L |
| Sodium dihydrogen phosphate (NaH₂PO₄.2H₂O) | 125 mg/L |
| HEPES | 6 g/L |
| | |
| L-alanine | 9 mg/L |
| L-asparagine | 13 mg/L |
| L-arginine.HCl | 126 mg/L |
| L-aspartate | 13 mg/L |
| L-cystein | 50 mg/L |
| L-glutamate | 200 mg/L |
| Glycine | 15 mg/L |
| L-hisitidine.HCl.H₂O | 40 mg/L |
| L-isoleucine | 52 mg/L |
| L-leucine | 52 mg/L |
| L-lysine.HCl | 72 mg/L |
| L-methionine | 15 mg/L |
| L-phenylalanine | 32 mg/L |
| L-proline | 15 mg/L |
| L-serine | 20 mg/L |
| L-threonine | 40 mg/L |
| L-tryptophan | 10 mg/L |
| L-tyrosine | 36 mg/L |
| L-valine | 46 mg/L |

### Vitamins

| | |
|---|---|
| Pantothenate | 4 mg/L |
| Choline | 4 mg/L |
| Folic acid | 4 mg/L |
| Nicotinamide | 4 mg/L |
| Pyridoxine | 4 mg/L |
| Riboflavine | 0.05 mg/L |
| Thiamine | 4 mg/L |
| Vitamin B₁₂ | 0.013 mg/L |

### Other components

| | |
|---|---|
| Phenol red | 15 mg/L |
| Nucleosides | 25mg/L each of adenosine, cytidine, guanosine and uridine. |
| Pyruvate | 220 mg/L |
| Essential fatty acids | 10 µg/L each of linoleic acid and linolenic acid |
| Non-essential fatty acids | 10 µg/L each of oleic acid palmitic acid, stearic acid |
| Cholesterol | 0.2 mg/L |

The basal medium is adjusted to an osmolarity of 0.36 either by dilution with sterile water (to reduce osmolarity) or by adding sodium chloride to increase osmolarity.

Before use, the medium is supplemented with the following components
- Dialysed horse serum 10% v/v
- Sodium selenite 10 µg/L
- Glutathione 0.5mg/L
- L-glutamine 10 mg/L
- Interleukin-3 4000 u/L

The basal medium is adjusted to an osmolarity of 0.36 either by dilution with sterile water (to reduce osmolarity) or by adding sodium chloride to increase osmolarity.

### Example 2: Example of a culture method

An example of a culture method is provided by the culture of the FDCPmix cell lines in the medium according to example 1, compared to culture in standard media.

### Description of the FDCPmix cell lines, known culture methods and methods of analysis:

FDCPmix cells are multipotential haematopoietic early progenitor cell lines, which have been studied extensively as a model of haematopoietic stem cells (Spooncer et al., 1993). FDCPmix cells have previously been cultured in a standard IMDM, DMEM or Fischer's medium supplemented with 20% horse serum (non-dialysed) and 100u/ml IL-3. Cell culture has previously been performed at 37°C in a controlled atmosphere of 5% CO₂ in air (about 20% O₂, 75 % N₂). Under these conditions the population consists of a mixture of multipotent (early) cells and differentiating (later) cells.

The proportion of multipotent cells in a given FDCPmix population can be assessed by dispersing a known number of cells into a medium containing the same components as that used for maintaining the FDCPmix cells, but made semi-solid by the inclusion methylcellulose or molten agarose. The early cells are capable of extensive self-renewal and form colonies over a period of 7 - 14 days of incubation at 37°C in an atmosphere of 5% CO₂ in air.

The differentiation potential of the cells in a given FDCPmix population is determined by removing them to a standard IMDM medium containing 10-20% fetal calf serum, 1-2u/ml IL-3, and additional growth factors supporting the development of myeloid cells (GM-CSF, G-CSF, M-CSF) or erythroid (erythropoietin) cells (Kan et al., 1993).

*Example of a culture method using the medium of the invention:* FDCPmix cells were placed into the medium described in example 1 at a density of 5 x 10⁴ cells /ml, and cultured in an atmosphere of 1% O₂, 5% CO₂, 94% N₂. Every 4 to 6 days, the number of live and dead cells was determined and the cell density adjusted to 5 x 10⁴ cells per ml either by centrifugation of the cells, removal of the supernatant medium and replacement with a suitable volume of fresh medium (days 4,8, 20, 24, 34, 38 and 42 of culture) or by simply adding a suitable amount of fresh medium to the culture (days 14 and 28 of culture).

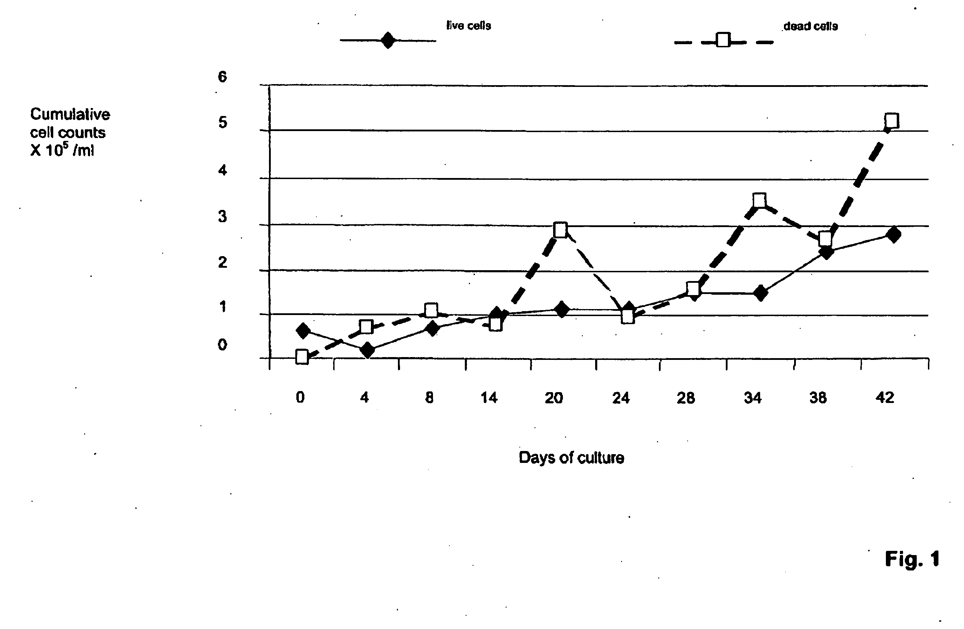

*Results:* As shown in **Fig 1**., the majority of cells in a FDCPmix population previously maintained in media containing glucose and inositol and then transferred into the medium deficient in glucose and inositol die within the first 4 days of culture, demonstrating that the medium is selective for a sub-population of the FDCPmix cells. Thereafter, the surviving cells proliferate, resulting in a steady increase in live cell number throughout the culture period.

This demonstrates that the cell population which survives in the medium deficient in glucose and inositol is capable of long term proliferation over a period of at least 6 weeks. Under the conditions of this experiment, the proliferating population produced a substantial proportion of cells which die, demonstrating that proliferation of those "early" cells independent of glucose and inositol produces not only more cells with the same properties, but also a substantial number of "later" cells which are dependent on glucose and/or inositol and are therefore eliminated from the culture.

### Example 3 Isolation and enrichment of stem cells from the umbilical cord blood

*Pre-enrichement:* The pre-enrichment uses methods established as standard practice in haematology research. Starting with 50 ml volume of human umbilical cord blood, the mononuclear cell fraction containing hematopoietic stem cells is purified away from other bone marrow components by density gradient centrifugation over Ficoll-Paque^{®} (non ionic synthetic polymer of sucrose) or a similar proprietary centrifugation medium. The mononuclear fraction is washed in phosphate-buffered saline solution, recovered by centrifugation. The subpopulation of mononuclear cells which express the surface antigen CD34 are isolated by a magnetic affinity approach using the MACS^{®} CD34 Kit (Miltenyi Biotech) under conditions recommended by the manufacturer. In short, the mononuclear cell population is resuspended at a concentration of 3 x 10⁸ cells per ml, incubated with magnetic beads carrying antibodies to the surface antigen CD34, then applied to a column arranged in a magnetic field, such that particle-bound (CD34⁺) cells are retained while non-bound cells are eluted and discarded. The CD34⁺ population is eluted from the column following removal from the magnetic field.

*Further enrichment in the medium of the invention:* The CD34⁺ population is transferred at a cell concentration of 1 x 10⁵ cells per ml into the medium of the invention supplemented with 10% dialysed autologous human serum, then incubated at 37°C in an atmosphere of 1% O₂, 5% CO₂ and 94% N₂.

After a period of three to seven days, the surviving cell population is enriched for stem cells and early progenitor cells and may be recovered by centrifugation.

### Example 4 Purification of non-leukaernic stem cells from leukaemic cells in a bone marrow sample from a patient with leukaemia

The CD34⁺ mononuclear cell fraction from a sample of human bone marrow is pre-enriched using the standard procedures of density gradient centrifugation and magnetic affinity described in example 3. The CD34⁺ population, which typically contains a mixture of both leukemic stem and progenitor cells and normal stem and progentior cells, is transferred at a cell concentration of 1 x 10⁵ cells per ml to the medium of the invention supplemented with 10% dialysed autologous human serum, then incubated at 37 °C in an atmosphere of 1% O₂, 5% CO₂ and 94% N₂.

After a period of three to seven days, the surviving cell population is enriched for normal stem and early progenitor cells and depleted for leukaemic stem and progenitor cells, and may be recovered by centrifugation.

### References

Hofmeister, C.C., Zhang, J., Knight, K.L., Le, P. And Stiff, P.J. (2007) Ex vivo expansion of umbilical cord blood stem cells for transplantation: growing knowledge from the hematopoietic niche. Bone Marrow Transplantation, 39 (1) pp11-23
Kan, O., Whetton, A.D. and Heyworth, C.M.(1993) Development of haematopoietic cells I liquid culture. In "Haemapoiesis a practical approach" ed.N.G. Testa and G. Molineux, Oxford University Press, Oxford. ISBN 0-19-963366-5 pp123-137.
Spooncer, E., Boettinger, D. and Dexter, T.M. (1993) Isolation and culture of factor-dependent haematopoietic cell lines. In "Haemapoiesis a practical approach" ed.N.G. Testa and G. Molineux, Oxford University Press, Oxford. ISBN 0-19-963366-5. pp107-121.
Wilson, A. and Trumpp, A. (2006) Bone-marrow haematopoietic-stem-cell niches. Nat Rev Immunol., 6, 93-106.

## Claims

1. Use of a cell culture medium containing less than 500 mg glucose/L and less than 500 µg inositol/L comprising
a basal medium provided as a diluted solution, a stock solution or powder containing less than 500 mg glucose/L and less than 500 µg inositol /L referring to the final diluted solution,
for culturing, amplifying, purifying and/or enriching somatic stem cells or germ line stem cells.

2. Use according to claim 1, whereas the basal medium contains less than 100 mg glucose/L and/or less than 100 µg inositol /L, referring to the final diluted solution.

3. Use according to claim 2, whereas the basal medium contains less than 10 mg glucose/L and/or less than 10 µg inositol /L, referring to the final diluted solution.

4. Use according to one of the claims 1 to 3, whereas the basal medium contains less than 200 mg/L L-glutamine and containing between 10 mg/L and 500 mg/L L-glutamate referring to the final diluted solution.

5. Use according to one of the claims 1 to 4, whereas the basal medium contains between 10 mg/L and 200 mg/L L-serine and between 5 mg/L and 100 mg/L glycine and/or between 5 mg/L and 200 mg/L L-cysteine, referring to the final diluted solution.

6. Use according to one of the claims 1 to 5, whereas the basal medium additionally contains between 1 mg/L and 1 g/L nucleosides or nucleoside precursors referring to the final diluted solution.

7. Use according to claim 6, wherein the basal medium contains between 1 mg/L and 50 mg/L each of adenosine, guanosine, cytidine and uridine.

8. Use according to one of the claim 1 to 7, wherein the cell culture medium is supplemented either by serum or a serum replacement component.

9. Use according to claim 8, whereas the serum replacement component comprises albumin, selenium, transferrin and essential fatty acids.

10. Use according to claim 8 or 9, whereas the glucose and inositol content in the serum is to be reduced by dialysis and/or enzymatic treatment.

11. Use according to one of the claims 8 to 10, whereas the cell culture medium is supplemented with growth factors supporting stem cell expansion, other activators and/or inhibitors of cellular signalling pathways.

12. Method for culturing, amplifying, purification and/or enrichment of somatic stem cells or germ line stem cells wherein cells are cultured *in vitro* in a cell culture medium as defined in one of the claims 1 to 11.

13. Method according to claim 12 wherein the cells are cultured in an atmosphere containing 0.1% to 5% O₂.

14. Method according to claim 12 or 13 wherein the stem cells are to be enriched from the blood and/or bone marrow.

15. Method according to claim 14 wherein the blood and/or bone marrow is to be isolated from a patient with cancer or leukaemia.

## Patentansprüche

1. Verwendung eines Zellkulturmediums, welches weniger als 500 mg Glukose/L und weniger als 500 µg Inositol/L enthält, umfassend
ein Basalmedium in der Form einer verdünnten Lösung, Stammlösung oder eines Pulvers, welches bezogen auf die endverdünnte Lösung weniger als 500 mg Glukose/L und weniger als 500 µg Inositol/L enthält,
für die Kultivierung, Vermehrung, Aufreinigung und/oder Anreicherung von somatischen Stammzellen oder Keimbahn-Stammzellen.

2. Verwendung nach Anspruch 1, wobei das Basalmedium bezogen auf die endverdünnte Lösung weniger als 100 mg Glukose/L und weniger als 100 µg Inositol/L enthält.

3. Verwendung nach Anspruch 2, wobei das Basalmedium bezogen auf die endverdünnte Lösung weniger als 10 mg Glukose/L und weniger als 10 µg Inositol/L enthält.

4. Verwendung nach einem der Ansprüche 1 bis 3, wobei das Basalmedium bezogen auf die endverdünnte Lösung weniger als 200 mg/L L-Glutamin und zwischen 10 mg/L und 500 mg/L L-Glutamat enthält.

5. Verwendung nach einem der Ansprüche 1 bis 4, wobei das Basalmedium bezogen auf die endverdünnte Lösung zwischen 10 mg/L und 200 mg/L L-Serin und zwischen 5 mg/L und 100 mg/L Glycin und/oder zwischen 5 mg/L und 200 mg/L L-Cystein enthält.

6. Verwendung nach einem der Ansprüche 1 bis 5, wobei das Basalmedium bezogen auf die endverdünnte Lösung zusätzlich zwischen 1 mg/L und 1 g/L Nukleoside oder Nukleosid-Präkursoren enthält.

7. Verwendung nach Anspruch 6, wobei das Basalmedium jeweils zwischen 1 mg/L and 50 mg/L Adenosin, Guanosin, Cytidin und Uridin enthält.

8. Verwendung nach einem der Ansprüche 1 bis 7, wobei das Zellkulturmedium mit Serum oder einer Serumersatzkomponente ergänzt ist.

9. Verwendung nach Anspruch 8, wobei die Serumersatzkomponente Albumin, Selen, Transferrin und essentielle Fettsäuren enthält.

10. Verwendung nach Anspruch 8 oder 9, wobei der Glukose- und Inositolgehalt im Serum durch Dialyse und/oder Enzymbehandlung reduziert wird.

11. Verwendung nach einem der Ansprüche 8 bis 10, wobei das Zellkulturmedium mit Wachstumsfaktoren, welche die Expansion der Stammzellen unterstützen, anderen Aktivatoren und/oder Inhibitoren von Zellsignalwegen ergänzt ist.

12. Verfahren zur Kultivierung, Vermehrung, Aufreinigung und/oder Anreicherung von somatischen Stammzellen oder Keimbahn-Stammzellen, bei dem die Zellen in einem Zellkulturmedium, wie in einem der Ansprüche 1 bis 11 definiert, *in vitro* kultiviert werden.

13. Verfahren nach Anspruch 12, bei dem die Zellen in einer Atmosphäre kultiviert werden, welche 0, 1 % to 5% O₂ enthält.

14. Verfahren nach Anspruch 12 oder 13, bei dem die Stammzellen aus dem Blut und/oder Knochenmark angereichert werden.

15. Verfahren nach Anspruch 14, bei dem das Blut und/oder Knochenmark aus einem Patienten mit Krebs oder Leukämie isoliert wird.

## Revendications

1. Utilisation d'un milieu de culture cellulaire contenant moins de 500 mg/l de glucose et moins de 500 µg/l d'inositol comprenant :
un milieu basal fourni sous forme d'une solution diluée, d'une solution mère ou d'une poudre contenant moins de 500 mg/l de glucose et moins de 500 µg/l d'inositol, par rapport à la solution diluée finale,
en vue de la mise en culture, de l'amplification, de la purification et/ou l'enrichissement de cellules souches somatiques ou de cellules souches de lignée germinale.

2. Utilisation selon la revendication 1, où le milieu basal contient moins de 100 mg/l de glucose et/ou moins de 100 µg/l d'inositol, par rapport à la solution diluée finale.

3. Utilisation selon la revendication 2, où le milieu basal contient moins de 10 mg/l de glucose et/ou moins de 10 µg/l d'inositol, par rapport à la solution diluée finale.

4. Utilisation selon l'une des revendications 1 à 3, où le milieu basal contient moins de 200 mg/l de L-glutamine et contenant entre 10 mg/l et 500 mg/l de L-glutamate, par rapport à la solution diluée finale.

5. Utilisation selon l'une des revendications 1 à 4, où le milieu basal contient entre 10 mg/l et 200 mg/l de L-sérine et entre 5 mg/l et 100 mg/l de glycine et/ou entre 5 mg/l et 200 mg/l de L-cystéine, par rapport à la solution diluée finale.

6. Utilisation selon l'une des revendications 1 à 5, où le milieu basal contient en plus entre 1 mg/l et 1 g/l de nucléosides ou de précurseurs de nucléosides, par rapport à la solution diluée finale.

7. Utilisation selon la revendication 6, où le milieu basal contient entre 1 mg/l et 50 mg/l de chaque composé parmi l'adénosine, la guanosine, la cytidine et l'uridine.

8. Utilisation selon l'une des revendications 1 à 7, où le milieu de culture cellulaire est ajouté soit à un sérum, soit à un composant de remplacement de sérum.

9. Utilisation selon la revendication 8, où le composant de remplacement de sérum comprend de l'albumine, du sélénium, de la transferrine et des acides gras essentiels.

10. Utilisation selon la revendication 8 ou 9, où la teneur en glucose et en inositol dans le sérum est réduite par une dialyse et/ou un traitement enzymatique.

11. Utilisation selon l'une des revendications 8 à 10, où le milieu de culture cellulaire se voit ajouter des facteurs de croissance supportant un développement de cellules souches, d'autres activateurs et/ou des inhibiteurs de voies de signalement cellulaire.

12. Procédé de mise en culture, d'amplification, de purification et/ou d'enrichissement de cellules souches somatiques ou de cellules souches de lignée germinale, où les cellules sont mises en culture *in vitro* dans un milieu de culture cellulaire selon l'une des revendications 1 à 11.

13. Procédé selon la revendication 12, dans lequel les cellules sont mises en culture dans une atmosphère contenant entre 0,1 % et 5 % d'O₂.

14. Procédé selon la revendication 12 ou 13, dans lequel les cellules souches sont enrichies à partir du sang et/ou de la moelle osseuse.

15. Procédé selon la revendication 14, où le sang et/ou la moelle osseuse sont isolés à partir d'un patient présentant un cancer ou une leucémie.
